# EUROPEAN PATENT SPECIFICATION

(11) **EP 1 625 559 B1**
(45) Date of publication and mention of the grant of the patent: **09.04.2008**
(21) Application number: 04730337.5
(22) Date of filing: 29.04.2004
(51) Int. Cl.: G08B 21/06, B60K 28/06

(54) **A DISTRESS SIGNALING SYSTEM, A BODY AREA NETWORK FOR ENABLING A DISTRESS SIGNALING, A METHOD FOR SIGNALING A CONDITION OF A DISTRESS AND A VEHICLE ARRANGED WITH A DISTRESS SIGNALING SYSTEM**
NOTFALLMELDESYSTEM, NOTFALLMELDESYSTEM ERMÖGLICHENDES KÖRPERLICHES NETZ, VERFAHREN ZUR MELDUNG EINER NOTLAGE UND FAHRZEUG AUSGESTATTET MIT EINEM NOTFALLMELDESYSTEM
SYSTEME DE SIGNALISATION DE DETRESSE, RESEAU CORPOREL PERMETTANT D'ACTIVER UNE SIGNALISATION DE DETRESSE, PROCEDE DE SIGNALISATION D'UN ETAT DE DETRESSE ET VEHICULE EQUIPE D'UN SYSTEME DE SIGNALISATION DE DETRESSE

(30) Priority: 08.05.2003 EP 03101270
(43) Date of publication of application: 15.02.2006
(73) Proprietor: Philips Intellectual Property & Standards GmbH, 20099 Hamburg (DE); Koninklijke Philips Electronics N.V., 5621 BA Eindhoven (NL)
(72) Inventor: MUEHLSTEFF, Jens c/o Philips Int. Prop.Stand.GmbH, 52066 Aachen (DE); REITER, Harald c/o Philips Int. Prop. Stand. GmbH, 52066 Aachen (DE); MONTVAY, Andras c/o Philips Int.Prop.Stand. GmbH, 52066 Aachen (DE); LAUTER, Josef c/o Philips Int. Prop. Stand. GmbH, 52066 Aachen (DE); SUCH, Olaf c/o Philips Int. Prop. Stand. GmbH, 52066 Aachen (DE); SCHMIDT, Ralf c/o Philips Int. Prop. Stand. GmbH, 52066 Aachen (DE); PERKUHN, Michael c/o Philips Int.Prop.Stand. GmbH, 52066 Aachen (DE); KOHLER, Fabian c/o Philips Int. Prop. Stand. GmbH, 52066 Aachen (DE)
(74) Representative: Volmer, Georg
(86) International application number: PCT/IB2004/001488
(87) International publication number: WO 2004/100100

(56) References cited:
- WO-A-00/13582
- GB-A- 2 375 645
- US-A- 4 706 072
- US-A- 5 724 024
- US-A- 5 853 005
- US-B1- 6 393 348

## Description

The invention relates to a distress signaling system for a seated passenger in a vehicle.
The invention still further relates to a method for signaling a condition of a distress of a seated passenger in a vehicle.
The invention still further relates to a vehicle comprising a distress signaling system.

A signaling system for a seated passenger in a vehicle is known from US 5,990, 795. The known signaling system is arranged to provide means of awakening a sleeping or unconscious operator of the vehicle by sounding an alarm with a recorded message within the cabin of the vehicle. For that purpose the known signaling system comprises monitoring means comprising a capacitance element arranged in a shoulder belt of a fastening system of a vehicle seat. The capacitance element is arranged to monitor a value of the capacitance, it being changed upon an event the operator of the vehicle falls into a sleep, which is usually followed by a nodding of the operator's head.

It is a disadvantage of this known signaling system that it provides means for monitoring a single manifestation of an abnormal operator's condition. This known system is not suitable for detecting a condition of a distress of the operator, for example a health-related abnormality or a condition of an anxiety, which can also potentially be harmful to the passenger. This known system also cannot detect a condition of a sleep or unconsciousness, when the head of the operator is not touching his chest.

WO 00/13582 A discloses a method of monitoring a subject in a vehicle seat, the method comprising acquiring a target signal from the subject with a piezoelectric transducer provided in or on the vehicle seat; processing the target signal to extract a cardiac signal and/or a respiratory signal; and outputting the cardiac and/or respiratory signal. In GB-A- 2 375 645, an apparatus for detecting drowsiness of a user is disclosed, the apparatus comprising a detector for detecting the instantaneous value of a metabolic function such as heart rate or body temperature of the user, comparison means for comparing the detected instantaneous value of the user's metabolic function with a stored value of that function representing the value in the sleeping state, and alarm indication means for generating an output signal when the detected value falls below a threshold value in the vicinity of the sleeping state value.

It is an object of the invention to provide a signaling system which is capable of detecting different kinds of a passenger's distress conditions, ranging from a condition of an anxiety to a condition of a medical emergency, while the passenger is being seated in the vehicle. This object is achieved by the subject matter of the independent claims.

A distress signaling system according to the invention comprises:
- monitoring means arranged in said vehicle to monitor a vital sign of the seated passenger by means of a sensor arranged to measure a signal representative of said sign;
- data processing means arranged to process the measured signal in order to yield data representative to a condition of said passenger;
- analysis means arranged to analyze said data in order to yield a condition-related parameter, said analysis means being further arranged to compare said condition-related parameter to a preset valid parameter and to generate a trigger signal upon an event said condition-related parameter exceeds said preset valid parameter;

The sensor comprising magnetic means arranged as a resonant circuit, said magnetic means being conceived to induce an oscillating magnetic field in a body volume of the passenger, said magnetic means being connectable to a power supply means, said data processing means being arranged to determine an amount of a power loss of said resonant circuit upon an application of said magnetic field to said body volume.

Preferably, the system comprises indicating means actuatable by the trigger signal, said indicating means being arranged to generate a feedback to said passenger.

The invention also consists of a method according to claim 10.

The technical measure of the invention is based on the insight that a condition of a distress is accompanied by a change in at least one vital sign of a person in question. Therefore, by monitoring a suitable vital sign and by carrying out a suitable analysis thereof, a reliable and versatile distress signaling system is obtained. It must be understood that under the definitions of the current invention, the term passenger is applicable to the operator of the vehicle and to any other seated person in the vehicle.

Preferably, the monitoring means is arranged in a direct vicinity of a seat of the vehicle or is integrated in the seat and/or a belt-fastening system. The monitoring means in the system according to the invention comprise a sensor arranged to monitor a vital sign by means of measuring a suitable signal. Preferably, a cardiac activity by means of measuring an ECG-signal and/or a respiration rate by means of acquiring a plethysmogram are being monitored. The sensor is further arranged to make available the measured signal to the data processing means, which is arranged to process the measured signal in order to yield data representative to a condition of the passenger.

For example, in case the cardiac activity of the passenger is monitored, the data processing means analyze an output signal from the sensor and deduce corresponding ECG spectra. The resulting ECG spectra are then forwarded by the data processing means to the data analysis means, which is arranged to analyze the spectra to yield a condition-related parameter. Examples of a suitable condition-related parameter are a heart-rate, a value of an amplitude of a selected peak in the ECG spectrum, or any other suitable characteristic deduced from the ECG spectrum. The analysis means is further arranged to compare the condition-related parameter to a preset valid parameter. An example of a suitable preset valid parameter is a threshold value of a heart-rate. It is also possible to prescribe a plurality of valid parameters, corresponding to a plurality of conditions of the passenger. For example, the system according to the invention can comprise suitable storage means, for example a memory unit, where a plurality of valid parameters are being stored for comparison purposes. Said plurality of valid parameters can comprise a first value of the valid parameter, corresponding to a first condition of the passenger, for example a condition of being asleep. Further, said plurality of valid parameters can comprise a second value of the valid parameter, corresponding to a second condition of the passenger, for example a condition of an anxiety. Still further, said plurality of valid parameters can comprise a third value of the valid parameter, corresponding to a third condition of the passenger, for example a condition of a non- life threatening medical abnormality. Still further, said plurality of valid parameters can comprise a forth value of the valid parameter, corresponding to a forth condition of the passenger, for example a condition of a medical emergency.

The analysis means of the system according to the invention is further arranged to compare the yielded condition-related parameter to the preset valid parameter. In case the yielded condition-related parameter exceeds the preset parameter, a trigger signal actuating the indicating means is generated. The indicating means is arranged to generate a feedback to the passenger upon a receipt of the trigger signal. An example of a suitable feedback is a replay of a prerecorded message, like a verbal message or an instruction or a musical melody. It is also possible that a feedback comprises a buzz-tone. Alternatively, the feedback can comprise an actuation of a cabin light of the vehicle or a suitable actuation of a seat under the passenger in question. Preferably, in case the passenger in question is a driver of the car, the seat is brought into a slightly vibrating state.

It is found to be advantageous that the indicating means is further arranged to control a climate control means of the vehicle upon a receipt of a trigger signal. In a situation when the seated passenger is experiencing a state of anxiety, it can be favorable to create a more pleasant environment, by correspondingly varying a cabin temperature of the vehicle, for example. In case it is detected that the seated passenger is suffering from a condition of a medical emergency, the indicating means is preferably arranged to actuate an alarming means of the vehicle upon a receipt of the trigger signal. In such case, for example, the external lights and/or an acoustic alarming means, for example, a horn of the vehicle can be actuated in order to attract an attention of possible bystanders. This feature is of particular advantage in case the seated passenger is a driver. This action is preferably followed by a controlling of the engine of the vehicle, for example by means of an actuation of a cruise control or any other suitable means.

It is found to be particularly advantageous to provide the monitoring means which are suited to cany-cut a substantially contact-less monitoring of a vital sign. The sensor according to the present embodiment comprises magnetic means which is arranged to induce an oscillating magnetic field in the body volume of the seated passenger upon an actuation of said sensor. The measuring principle is based on the Faraday's law. As biological tissue is a conductor, the oscillating magnetic filed induces eddy currents in the body of the passenger. The density of the induced eddy currents is proportional to the conductivity of the volume. The induced eddy currents generate a secondary magnetic field, pointing in an opposite direction with respect to a primary magnetic field. In accordance with the Faraday's law the secondary magnetic field induces an electromotive force in the primary coil, the phase of said force being 180 degrees shifted with respect to the direction of a driving current. The conductive body can thus be represented as a resistive load to the driving current. By measuring a power loss of the resonant circuit, a conclusion about the conductivity of the volume under investigation can be drawn. Because the internal impedance of the conductive body is finite, any change in load resistance due to a change in the conductivity will cause an amplitude of the measured signal to vary. When the characteristics of the primary resonant circuit are known, the conductivity of the volume under investigation can thus be determined. In a human body the conductive medium is blood. Therefore, a determination of a blood flow in a volume located in a vicinity of a resonant circuit can be used for monitoring purposes. This is particularly suitable for cardiac applications.

Alternatively, it is possible to monitor a respiration rate, as during inhalation the conductivity of thorax decreases due to an air inflow.

In a further embodiment of a system according to the invention the magnetic means comprise a coil with a loop of a conductor, said loop being integrated into a shoulder portion of a set belt.

It is found to be of a particular advantage to integrate the magnetic means into the shoulder portion of the seat belt. The shoulder portion of the seat belt is defined as a loop of the seat belt, which transverses a thorax region of the passenger. Preferably, the loop of the conductor is positioned about 50 cm from a fixating means of the belt, this ensuring that the coil will be positioned substantially near a heart or a stomach of the seated passenger. Alternatively, the magnetic means can be adjustably attached to the shoulder portion of the seat belt, so that the seated passenger can customize their position with respect to his body. Preferably, the magnetic means are actuated upon a fastening of the seat belt, the belt fastening means being provided with a suitable wiring leading to a suitable battery of the vehicle.

Alternatively, the magnetic means of the system according to the invention can be integrated in a back support portion of the seat in a region, substantially corresponding to a thorax region of the seated passenger. In this case, the magnetic means can comprise a permanent wiring to a battery of the vehicle, and can be actuated upon a start-up of the vehicle's engine.

In a still further embodiment of the system according to the invention the sensor comprises an RF-transmitter unit and an RF-receiver unit, said RF-transmitter unit and said RF-receiver unit being conceived to be arranged under operating conditions in a spatial relation so that the body volume is located substantially therebetween.

This alternative embodiment enables a measurement of a blood flow in the body volume located between the RF-transmitter unit and the RF-receiver unit. The measuring principle is based on inductive coupling of two coils which varies by a conducting medium in between the coils. A change in a value of the induced voltage at the RF-receiver is a measure of the conductivity of the body volume.

A vehicle according to the invention comprises the distress signaling system, said system comprising: monitoring means arranged in said vehicle to monitor a vital sign of the seated passenger by means of a sensor arranged to measure a signal representative of said sign; data processing means arranged to process the measured signal in order to yield data representative to a condition of said passenger; analysis means arranged to analyze said data in order to yield a condition-related parameter, said analysis means being further arranged to compare said condition-related parameter to a preset valid parameter and to generate a trigger signal upon an event said condition-related parameter exceeds said preset valid parameter; indicating means actuatable by the trigger signal, said indicating means being arranged to generate a feedback to said passenger.

In a preferred embodiment of the vehicle according to the invention, said vehicle comprises electronic means arranged to customize a setting of a vehicle in order to yield a preferred setting, said preferred setting being selectable from a plurality of pre-stored valid settings, said electronic means being further arranged to select a valid parameter in accordance with the selected preferred setting.

It is found particularly advantageous to personalize the functioning of the distress signaling system, for example, in case the vehicle is being operated by different persons at different times, said vehicle comprising per se known means to customize the vehicle settings, like a position of the seat, a cabin climate, etc.

It is advantageous to arrange electronic means to select a valid parameter in accordance with the selected preferred setting. For example, in case a car is driven by a healthy female and a male with a cardiac condition, the valid parameters of the female differ from the valid parameters of the male. Thus, in order to enable a precise functioning of the distress system personal valid parameters are downloaded from a database for each individual and are used as reference values as long as the passenger remains seated in the vehicle.

These and other aspects of the invention will be discussed in more detail with reference to figures.
Fig. 1 presents a schematic view of an embodiment of the distress signaling system according to the invention.
Fig. 2 present a schematic view of an embodiment of a sensor comprising magnetic means.
Fig. 3 presents a schematic front view of an embodiment of a sensor being integrated into a shoulder portion of a seat belt of the vehicle.
Fig. 4 presents a schematic view of a sensor comprising an RF- transmitter unit and an RF-receiver unit.

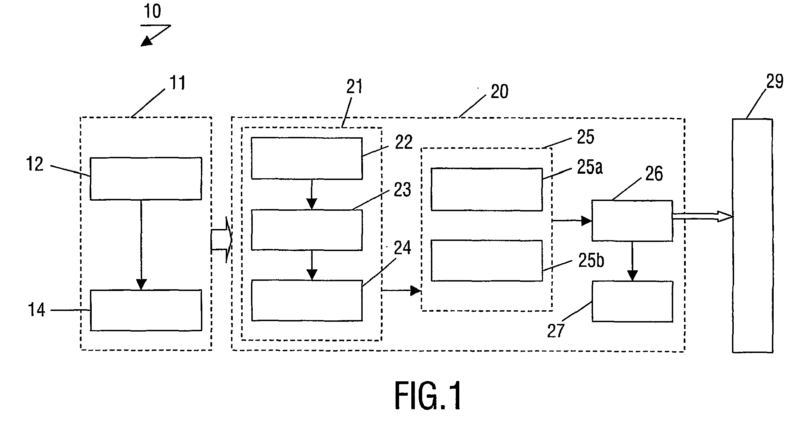

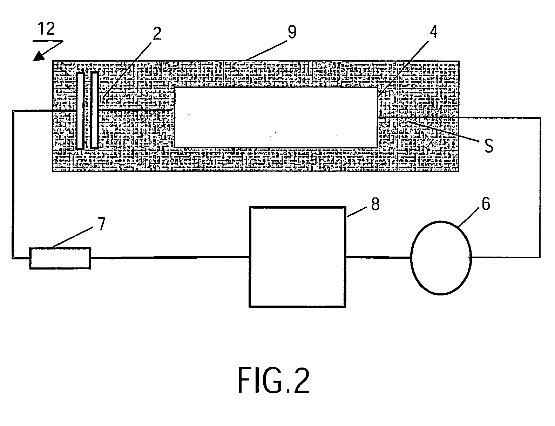

Fig. 1 presents a schematic view of an embodiment of the distress signaling system for a seated passenger according to the invention. The distress signaling system 10 comprises monitoring means 11 arranged in a vehicle in order to monitor a vital sign of the seated passenger by means of a sensor 12 arranged to measure a signal representative to said sign. The sensor 12 preferably comprise a resonant circuit (not shown) arranged in a vicinity of the body of the passenger to pick- up a signal characteristic of the targeted vital sign, for example a signal related to a blood flow. The sensor 12 can also be arranged to comprise a further magnetic means arranged as a further resonant circuit. The further magnetic means are conceived to induce an oscillating magnetic field in a further body volume of the seated passenger, for example to obtain a reference signal. Additionally, the sensor 12 can comprise a temperature meter, a blood pressure meter or any other suitable sensor. An operation of the sensor 12 will be discussed in more detail with reference to Fig. 2. Additionally, the monitoring means 11 can comprise a data pre-processing unit 14 arranged to carry-out a suitable pre-processing of the measured signal. The monitoring means 11 are preferably arranged to perform a continuous monitoring of the vital sign of the passenger and are further arranged to provide a corresponding signal, for example by means of a wire-less communication, to the front-end electronics 20 of the system 10. The front-end electronics 20 is arranged to analyze the signal from the monitoring means 11. For that purpose the front-end electronics 20 comprises a data processing means 21 arranged to process the measured signal in order to yield data representative to a condition of the passenger. The data processing means 21 comprises a preamplifier 22, an analogue processing circuit 22 and an ADC unit 23. The data processing means 21 forwards the corresponding data to the data analysis means 25, arranged to analyze said data in order to yield a condition-related parameter. For example, in case the sensor 12 is arranged to monitor a cardiac activity of the passenger, the resulting data comprises an electrocardiogram. In this case the data analysis means 25 is arranged to determine a corresponding heart-rate of the passenger. In case the sensor 12 is arranged to monitor a respiration rate of the passenger, the resulting data comprises an inhalation-exhalation curve. In this case the analysis means 25 is arranged to determine the respiration rate of the passenger. The determination of the corresponding rates can be done using a per se known computation algorithms (not shown). The thus determined condition-related parameter is written into a first look-up table 25a. The data analysis means 25 is further arranged to compare said condition-related parameter to a preset valid parameter stored in a second look-up table 25b. Preferably, the second look-up table 25b comprises a plurality of valid parameters. For example, a first valid parameter can be set to a condition of an anxiety of the passenger, a second valid parameter can be set to a condition of a minor medical abnormality and a further valid parameter can be set to a condition of a medical emergency. It is also possible that the look-up table 25b comprises a gradual conditioning of the valid parameter, given, for example by an analytical expression. In case the data analysis means detects that the condition-related parameter exceeds a preset valid parameter, it generates a trigger signal to actuate the indicating means 26. The indicating means 26 receive the trigger signal and generate a suitable feedback to the passenger. An example of a suitable feedback is a textual message of a user-interface 27. Alternatively, the user-interface can comprise a loud speaker (not shown) arranged to replay a prerecorded message or a musical tone.

Additionally, the indicating means 26 can be arranged to control a vehicle control system 29, in order to alter a cabin environment, or to actuate an alarming means of the vehicle, like a acoustic alarm and/or the emergency lights. Also, an engine of the vehicle can be controlled, for example to set the vehicle into a full stop, for example in case the driver of the vehicle is suffering from a medical emergency.

Fig. 2 presents a schematic view of an embodiment of a sensor 12 comprising magnetic means with a resonant circuit 2,4, 7. The resonant circuit comprises a resistor 7 consecutively connected to a capacitance 2 and a coil 4. Power supply means 8 energise the resonant circuit 7, 2, 4 so that an oscillating magnetic field (not shown) is produced. The sensor can be arranged with its dedicated power supply means, or alternatively be energised by a battery unit of the vehicle (not shown). The signal S from the resonant circuit 7, 2, 4 is detected by an amperemeter 6. The power loss experienced by the resonant circuit due to an electromagnetic interaction with a conductive body (not shown) is reflected in a change in the magnitude of the signal S.

By detecting the signal S the power loss by the resonant circuit is determined. In case the relation between the absolute value of the power loss and the signal S is known, the conductive characteristics of the volume being investigated can be determined. In order to ensure a constant power load, the resonant circuit preferably is enabled with a feedback loop (not shown). The feedback loop is preferably arranged so that the voltage controlling the amplitude of the resonant circuit is proportional to the RF power delivered by the resonant circuit. The resonant circuit is integrated into an insulating fabric carrier 9. Preferably, the conductors forming the coil 4 are interwoven with threads of fabric 9. In the simplest embodiment the resonant circuit comprises a single coil 4 with a single loop. In more sophisticated embodiments it is possible to design the resonator circuit with a plurality of coils comprising a plurality of loops. Preferably, the insulating fabric 9 is a part of a shoulder portion of a seat belt of the vehicle or a part of a back-support portion of the seat.

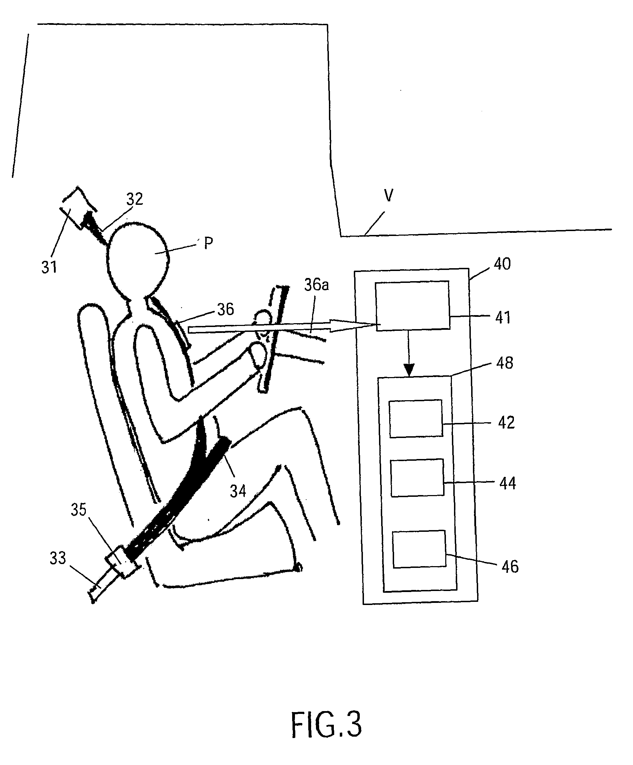

Fig. 3 presents a schematic front view of an embodiment of a sensor being integrated into a shoulder portion of a seat belt of the vehicle. The passenger P assumes a seated position and fastens the seat belt, comprising a shoulder portion 32 and a lower portion 34. The seat belt is fastened to the vehicle V by means of a fastening means 31, 33, respectively. It must be noted that a term vehicle under current definitions is applicable to a car, a train, a boat, an airplane, etc. The vehicle V is provided with a sensor 36 arranged to monitor a vital sign of the seated passenger. In a first embodiment the sensor 36 comprises magnetic means as is discussed with reference to Fig. 2 and is integrated into a shoulder portion 32 so that when the belt is fastened, the sensor will lie in a region near thorax of the passenger P. In this case a monitoring of a cardiac activity and a respiration rate of the passenger can be enabled.

The sensor 36 can be powered by a battery unit (not shown) of the vehicle, corresponding wiring being integrated into the shoulder portion of the seat belt, an electrical connection thereto 35 being integrated into the belt fastening means 33.

Preferably, the powering of the sensor 36 is actuated upon a fastening of the belt fastening means 33. The sensor 36 provides a signal representative to the vital sign of the passenger to the data processing means (not shown), which is preferably integrated into a dashboard 40 of the vehicle V. Preferably, a communication between the sensor 36 and the data processing means is enabled by means of a wire-less signaling, indicated by an arrow 36a. Upon an event a trigger signal (not shown) to the indicating means is actuated, the indicating means 41 generate a suitable feedback to the passenger P. Preferably, the indicating means send a control signal to a central processing unit 48 of the vehicle, said control signal being arranged to control an alarming means 42 of the vehicle, an engine of the vehicle 44 and a climate control means 46 of the vehicle.

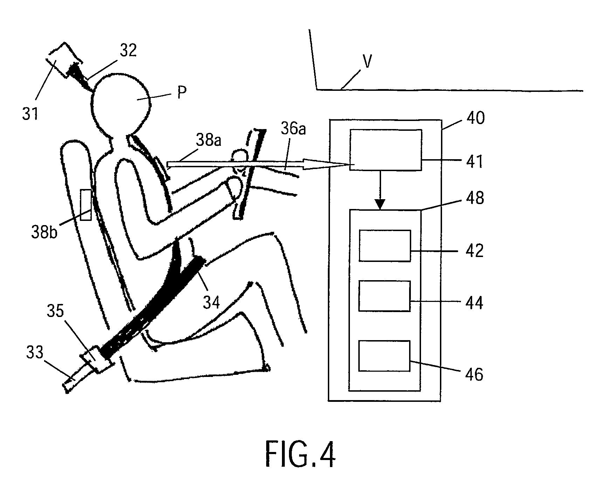

Fig. 4 presents a schematic view of a sensor comprising an RF- transmitter unit and an RF-receiver unit. The RF-transmitter unit 38b is preferably integrated into a back support portion of a seat of the vehicle V. The RF-receiver unit 38a is preferably integrated into a shoulder portion 32 of the seat belt of the seat of the vehicle V. The RF-transmitter unit is arranged to transmit a suitable RF-power to transverse a thorax region of the seated passenger. The measurement principle is as follows. The RF-transmitter unit comprises a first magnetic coil and the RF-receiver unit comprises a second magnetic coil, which are inductively coupled. An oscillating magnetic field which is generated by the first magnetic coil induces a voltage in the second magnetic coil. The induced voltage in the second magnetic coil varies in time if the conductivity of a medium between the first magnetic coil and the second magnetic coil (thorax, heart) Therefore, time variations of the conductivity in a body volume which can be caused e. g. by a heart activity and/or respiration influence the induced voltage in the second coil.

Based on that principle a variation of a received signal by the RF-receiver unit provides information on a cardiac and/or pulmonary activity of the seated passenger P. The RF-transmitter unit 38b is preferably provided with a stationary wiring to a battery unit (not shown) of the vehicle V. The RF-receiver unit 38a is preferably energized by the battery unit of the vehicle V upon a fastening of the belt fastening means 33, a corresponding electrical wiring 35 being integrated into the belt fastening means 33. The functioning of other units, shown in the Fig. 4 is substantially the same to the functioning of the corresponding units, as is discussed with reference to Fig. 3.

## Claims

1. A distress signaling system (10) for a seated passenger (P) in a vehicle (V), said system comprising:
- monitoring means (11) arranged in said vehicle to monitor a vital sign of the seated passenger by means of a sensor (12) arranged to measure a signal (S) representative of said sign;
- analysis means (25) arranged to analyze said signal (S) in order to yield a condition-related parameter (25a), said analysis means being further arranged to derive from said condition-related parameter a trigger signal by comparison with a preset valid parameter,
**characterized in that**
- the sensor (12) comprises a resonant circuit, said circuit being conceived to induce an oscillating magnetic field in a body volume of the passenger and data processing means being arranged to determine an amount of a power loss of said resonant circuit upon interaction of said magnetic field with said body volume.

2. A system according to claim 1, **characterized in that** the system further comprises indicating means (26) actuatable by the trigger signal, said indicating means being arranged to generate a feedback to said passenger.

3. A system according to claim 1, **characterized in that** the resonant circuit comprises a coil (4) with a loop of a conductor, said loop being integrated into a shoulder portion of a seatbelt (34) of the vehicle.

4. A system according to claim 1, **characterized in that** the resonant circuit comprises a coil (4) with a loop of a conductor, said loop being integrated into a back support portion of a seat of the vehicle.

5. A system according to claim 1, **characterized in that** the sensor comprises an RF-transmitter (38b) unit and an RF-receiver unit (38a), said RF-transmitter unit and said RF-receiver unit being conceived to be arranged under operating conditions in a spatial relation so that the body volume is located substantially therebetween.

6. A system according to claim 2, **characterized in that** the indicating means (26) comprises a loudspeaker arranged to generate an audio feedback to the passenger.

7. A system according to claim 6, **characterized in that** the indicating means (26) is further arranged to control a climate control means of the vehicle upon a receipt of a trigger signal.

8. A system according to claim 2, **characterized in that** the indicating means (26) is further arranged to actuate an alarming means (42) of the vehicle upon a receipt of the trigger signal.

9. A system according to claim 8, **characterized in that** the indicating means (26) is further arranged to control an engine (44) of the vehicle upon a receipt of the trigger signal.

10. A method of signaling a condition of distress of a seated passenger in a vehicle, said method comprising the steps of :
- monitoring a vital sign of the seated passenger in said vehicle by means of a sensor integrated in said vehicle, said sensor being arranged to measure a signal representing said sign by inducing an oscillating magnetic field by means of a resonant circuit in a body volume of the passenger and determining an amount of a power loss of said resonant circuit upon interaction of said magnetic field with said body volume;
- analyzing said signal in order to yield a condition-related parameter;
- comparing said condition-related parameter to a preset valid parameter;
- generating a feedback to said passenger upon an event said condition-related parameter deviates from the preset valid parameter with a preset amount.

11. A method according to claim 10, said method further comprising the step of actuating an alarming means of the vehicle.

12. A method according to claim 10, said method further comprising the step of controlling an engine of the vehicle.

13. A vehicle comprising a distress signaling system according to any of the preceding claims 1 to 9.

14. A vehicle according to claim 13, **characterized in that** the vehicle comprises electronic means arranged to customize a setting of a vehicle in order to yield a preferred setting, said preferred setting being selectable from a plurality of pre-stored valid settings, said electronic means being further arranged to select a valid parameter in accordance with the selected preferred setting.

## Patentansprüche

1. Notfallmeldesystem (10) für einen sitzenden Insassen (P) eines Fahrzeugs (V), wobei das genannte System Folgendes umfasst:
- Überwachungsmittel (11), die in dem genannten Fahrzeug angeordnet sind, um ein Vitalzeichen des sitzenden Insassen mit Hilfe eines Sensors (12) zu überwachen, der dafür ausgelegt ist, ein das genannte Vitalzeichen darstellendes Signal (S) zu messen;
- Analysemittel (25), die dafür ausgelegt sind, das genannte Signal (S) zu analysieren und einen zustandsbezogenen Parameter (25a) zu liefern, wobei die genannten Analysemittel weiterhin dafür ausgelegt sind, durch den Vergleich mit einem vorgegebenen gültigen Parameter ein Auslösesignal von dem genannten zustandsbezogenen Parameter abzuleiten,
**dadurch gekennzeichnet, dass**
- der Sensor (12) einen Schwingkreis umfasst, wobei der Schwingkreis so konzipiert ist, dass ein oszillierendes Magnetfeld in ein Körpervolumen des Insassen induziert wird, und Datenverarbeitungsmittel dafür ausgelegt sind, ein Ausmaß eines Energieverlusts des genannten Schwingkreises bei Interaktion des genannten Magnetfeldes mit dem genannten Körpervolumen zu bestimmen.

2. System nach Anspruch 1, **dadurch gekennzeichnet, dass** das System weiterhin durch das Auslösesignal betätigbare Meldemittel (26) umfasst, wobei die genannten Meldemittel dafür ausgelegt sind, eine Rückmeldung an den genannten Insassen zu erzeugen.

3. System nach Anspruch 1, **dadurch gekennzeichnet, dass** der Schwingkreis eine Spule (4) mit einer Leiterschleife umfasst, wobei die Schleife in einen Schulterteil eines Sicherheitsgurtes (34) des Fahrzeugs integriert ist.

4. System nach Anspruch 1, **dadurch gekennzeichnet, dass** der Schwingkreis eine Spule (4) mit einer Leiterschleife umfasst, wobei die genannte Schleife in den Rückenteil eines Fahrzeugsitzes integriert ist.

5. System nach Anspruch 1, **dadurch gekennzeichnet, dass** der Sensor eine HF-Sendeeinheit (38b) und eine HF-Empfangseinheit (38a) umfasst, wobei die genannte HF-Sendeeinheit und die genannte HF-Empfangseinheit so konzipiert sind, dass sie unter Betriebsbedingungen in einem räumlichen Verhältnis so angeordnet werden können, dass sich das Körpervolumen im Wesentlichen zwischen ihnen befindet.

6. System nach Anspruch 2, **dadurch gekennzeichnet, dass** die Meldemittel (26) einen Lautsprecher umfassen, der dafür ausgelegt ist, eine akustische Rückmeldung an den Insassen zu erzeugen.

7. System nach Anspruch 6, **dadurch gekennzeichnet, dass** die Meldemittel (26) weiterhin dafür ausgelegt sind, auf den Empfang eines Auslösesignals hin ein Klimaregelungsmittel des Fahrzeugs zu steuern.

8. System nach Anspruch 2, **dadurch gekennzeichnet, dass** die Meldemittel (26) weiterhin dafür ausgelegt sind, auf den Empfang des Auslösesignals hin ein Alarmierungsmittel (42) des Fahrzeugs zu betätigen.

9. System nach Anspruch 8, **dadurch gekennzeichnet, dass** die Meldemittel (26) weiterhin dafür ausgelegt sind, auf den Empfang des Auslösesignals hin einen Motor (44) des Fahrzeugs zu steuern.

10. Verfahren zum Melden einer Notlage eines sitzenden Insassen in einem Fahrzeug, wobei das genannte Verfahren die folgenden Schritte umfasst:
- Überwachen eines Vitalzeichens des sitzenden Insassen in dem genannten Fahrzeug mit Hilfe eines in das genannte Fahrzeug integrierten Sensors, wobei der genannte Sensor dafür ausgelegt ist, ein das genannte Vitalzeichen darstellendes Signal (S) zu messen, indem er mit Hilfe eines Schwingkreises ein oszillierendes Magnetfeld in ein Körpervolumen des Insassen induziert und ein Ausmaß eines Energieverlusts des genannten Schwingkreises bei Interaktion des genannten Magnetfelds mit dem genannten Körpervolumen bestimmt;
- Analysieren des genannten Signals, um einen zustandsbezogenen Parameter zu liefern;
- Vergleichen des genannten zustandsbezogenen Parameters mit einem vorgegebenen gültigen Parameter;
- Erzeugen einer Rückmeldung an den genannten Insassen, falls der genannte zustandsbezogene Parameter um einen vorgegebenen Betrag von dem genannten gültigen Parameter abweicht.

11. Verfahren nach Anspruch 10, wobei das genannte Verfahren weiterhin den Schritt des Betätigens eines Alarmierungsmittels des Fahrzeugs umfasst.

12. Verfahren nach Anspruch 10, wobei das genannte Verfahren weiterhin den Schritt des Steuerns eines Fahrzeugmotors umfasst.

13. Fahrzeug mit einem Notfallmeldesystem nach einem der vorhergehenden Ansprüche 1 bis 9.

14. Fahrzeug nach Anspruch 13, **dadurch gekennzeichnet, dass** das Fahrzeug elektronische Mittel umfasst, die dafür ausgelegt sind, eine Einstellung eines Fahrzeugs bedarfsmäßig anzupassen, um eine bevorzugte Einstellung zu erhalten, wobei die bevorzugte Einstellung aus einer Vielzahl von zuvor gespeicherten gültigen Einstellungen ausgewählt werden kann, wobei die genannten elektronischen Mittel weiterhin dafür ausgelegt sind, einen gültigen Parameter gemäß der ausgewählten bevorzugten Einstellung zu wählen.

## Revendications

1. Système de signalisation de détresse (10) pour un passager assis (P) dans un véhicule (V), ledit système comprenant :
- un moyen de surveillance (11) monté dans ledit véhicule afin de surveiller un signe vital du passager assis au moyen d'un capteur (12) conçu pour mesurer un signal (S) représentatif dudit signe;
- un moyen d'analyse (25) conçu pour analyser ledit signal (S) afin de produire un paramètre (25a) lié à une condition, ledit moyen d'analyse étant en outre conçu pour dériver dudit paramètre lié à une condition un signal de déclenchement suite à une comparaison avec un paramètre valide fixé au préalable, **caractérisé en ce que**
- le capteur (12) comprend un circuit résonnant, ledit circuit étant conçu pour induire un champ magnétique oscillant dans un volume corporel du passager, et le moyen de traitement de données étant conçu pour déterminer une quantité de perte de puissance dudit circuit résonant suite à une interaction dudit champ magnétique avec ledit volume corporel.

2. Système suivant la revendication 1, **caractérisé en ce que** le système comprend en outre un moyen d'indication (26) pouvant être actionné par le signal de déclenchement, ledit moyen d'indication étant conçu pour générer une rétroaction pour ledit passager.

3. Système suivant la revendication 1, **caractérisé en ce que** le circuit résonant comprend une bobine (4) avec une boucle d'un conducteur, ladite boucle étant intégrée dans une partie d'épaule d'une ceinture de sécurité (34) du véhicule.

4. Système suivant la revendication 1, **caractérisé en ce que** le circuit résonant comprend une bobine (4) avec une boucle d'un conducteur, ladite boucle étant intégrée dans une partie formant dossier d'un siège du véhicule.

5. Système suivant la revendication 1, **caractérisé en ce que** le capteur comprend une unité formant émetteur RF (38b) et une unité formant récepteur RF (38a), ladite unité formant émetteur RF et ladite unité formant récepteur RF étant conçues pour présenter, en conditions de fonctionnement, une relation spatiale, de sorte que le volume corporel se trouve sensiblement entre celles-ci.

6. Système suivant la revendication 2, **caractérisé en ce que** le moyen d'indication (26) comprend un haut-parleur conçu pour générer une rétroaction audio pour le passager.

7. Système suivant la revendication 6, **caractérisé en ce que** le moyen d'indication (26) est en outre conçu pour commander un moyen de régulation de climatisation du véhicule après réception d'un signal de déclenchement.

8. Système suivant la revendication 2, **caractérisé en ce que** le moyen d'indication (26) est en outre conçu pour actionner un moyen donnant l'alarme (42) du véhicule après réception du signal de déclenchement.

9. Système suivant la revendication 8, **caractérisé en ce que** le moyen d'indication (26) est en outre conçu pour commander un moteur (44) du véhicule après réception du signal de déclenchement.

10. Procédé de signalisation d'une condition de détresse pour un passager assis dans un véhicule, ledit procédé comprenant les étapes suivantes :
- surveiller un signe vital du passager assis dans ledit véhicule au moyen d'un capteur intégré dans ledit véhicule, ledit capteur étant conçu pour mesurer un signal représentatif dudit signe en induisant un champ magnétique oscillant au moyen d'un circuit résonant dans un volume corporel du passager, et pour déterminer une quantité de perte de puissance dudit circuit résonant suite à une interaction dudit champ magnétique avec ledit volume corporel;
- analyser ledit signal afin de produire un paramètre (25a) lié à une condition;
- comparer ledit paramètre lié à une condition à un paramètre valide fixé au préalable, et
- générer une rétroaction pour ledit passager suite à un événement pour lequel ledit paramètre lié à une condition est différent dudit paramètre valide fixé au préalable à raison d'une quantité fixée au préalable.

11. Procédé suivant la revendication 10, ledit procédé comprenant en outre l'étape pour actionner un moyen donnant l'alarme du véhicule.

12. Procédé suivant la revendication 10, ledit procédé comprenant en outre l'étape pour commander un moteur du véhicule.

13. Véhicule comprenant un système de signalisation de détresse suivant l'une quelconque des revendications 1 à 9 précédentes.

14. Véhicule suivant la revendication 13, **caractérisé en ce que** le véhicule comprend un moyen électronique prévu pour personnaliser un réglage d'un véhicule afin de produire un réglage préféré, ledit réglage préféré pouvant être sélectionné par une pluralité de réglages valides stockés au préalable, ledit moyen électronique étant en outre conçu pour sélectionner un paramètre valide conformément au réglage préféré sélectionné.
